# EUROPEAN PATENT SPECIFICATION

(11) **EP 1 023 035 B1**
(45) Date of publication and mention of the grant of the patent: **05.06.2002**
(21) Application number: 97950354.7
(22) Date of filing: 17.10.1997
(51) Int. Cl.: A61K 7/16

(54) **STOMATIC COMPOSITION**
MUNDPFLEGEZUBEREITUNG
COMPOSITION BUCCALE

(43) Date of publication of application: 02.08.2000
(73) Proprietor: Zakrytoe Aktsionernoe Obschestvo OSTIM, Moscow, 119034 (RU)
(72) Inventor: RUDIN, Vsevolod Nikolaevich, Moscow, 125502 (RU); ZUEV, Vladislav Petrovich, Moscow, 123426 (RU); KOMAROV, Vladimir Fedorovich, Moscow, 115582 (RU); MELIKHOV, Igor Vitallevich, Moscow, 117331 (RU); MINAEV, Vladimir Vasillevich, Moscow, 121248 (RU); ORLOV, Andrei Yurlevich, Moscow, 121357 (RU); MISHIN, Anatoly Aleksandrovich, Moscow, 117421 (RU); BOZHEVOLNOV, Viktor Evgenievich, Moscow, 109004 (RU)
(74) Representative: Herden, Andreas F.
(86) International application number: IB9701634
(87) International publication number: WO9920237

(56) References cited:
- EP-A- 0 664 133
- EP-A- 0 786 245
- WO-A-98/18719

## Description

### Description

### Field of the Invention

This invention relates to the field of medicine, and in particular to the field of stomatology and may be used for preventive treatment and curing of caries, parodenitis and paradentosis.

### Prior art

For these above-captioned purposes, stomatic compositions comprising hydroxyapatite (HA) have found an extensive application in the stomatologic practice.

There are certain compositions having a favourable effect including synthetic HA containing 92 to 97% Ca₁₀(PO₄)₆(OH)₂, 3 to 6% H₂O and 0,3% CaCO₃ with an average particle size of 1 to 15 µm.

Such a stomatic composition, for instance, according to Patent EP 0344832 cl. A61K 7/16, comprises save the stated HA, water-soluble casein material or sodium trimetaphosphate, as an anti-caries agent and also other well known ingredients which depend upon the forms of the product manufactured, such as various humectants, binding thickeners, surfactants, flavouring agents.

The known stomatic composition (EP 03442746 cl. A61K 7/18, publ.23.11.89) supplementary includes a fluorine-containing compound in the form of NaP or sodium monophluorphosphate as an anti-caries agent.

The amount of HA present in the stomatic composition is in the range of 1 to 50%, usually 2 to 20% by weight of the stomatic composition. The stomatic composition comprises some other ingredients: humectants, thickeners, surfactants and flavouring agents commonly known to those skilled in the art in all formulations of such products.

However, the stomatic compositions stated possess a relatively poor anti-caries effect and is not useful in the preventive medicine and in the treatment of inflammatory-destructive diseases of paradentium tissues.

From document EP-A-0664133 a preparation for stimulating growth in bone tissue based on hydroxyapatite with a particle size of 0,015 µm to 0,06 µm is known. However, that preparation is not intended to be used for stomatic applications.

A dental composition containing hydroxyapatite having a particle size from 0,05 µm to 1,0 µm is known from document EP-A-0786245. However, no specific shape of the particles is disclosed in document EP-A-0786245.

### Disclosure of the invention

It is an object of the present invention to create a stomatic composition comprising compounds capable to cure microdefects of the basic substance of the dental enamel to combat caries developing (e.g. to provide an anti-caries activity) and to prevent the spread of such inflammable-destructive diseases of paradentium tissues as paradenitis and paradentosis, and also compounds capable to stimulate reparative osteogenesis processes and possessing high bioactivity and specific pharmacological activity.

It is a further object of the invention to create a stomatic composition being identic to the basic substance of the dental enamel in its substance contents and crystalline parameters, as the acid formed in the materials covering dental surfaces causes destruction of mineral hydroxyapatite out of which enamel is composed and has a result due to which calcium ion loss occurs.

The task surprisingly has been solved in a composition as defined in claim 1.

A preferred composition having a more pronounced effect in view of the improvements obtained according to the invention comprises particles of hydroxyapatite with an average particle size in length (l), width(d) and thickness(h) of about l = 0,06 µm +/- 50 %, d = 0,015 µm +/- 50 % and h = 0,005 µm +/- 50 %.

A most preferred composition having a surprisingly superior effect in view of the improvements obtained according to the invention comprises particles of hydroxyapatite with an average particle size in length (l), width(d) and thickness(h) of 1 about 0,06 µm, d about 0,015 µm, h about 0,005 µm.

Being introduced into the composition, HA possesses osteo-reparative properties and contains preferably about 100% Ca₁₀(PO₄)₆(OH)₂.

The specific surface of HA used in the composite advantageously is 100 to 150 m²/g.

The amount of HA present in the oral composition of the present invention is in the range of 0,1% to 50%, preferably from 0,1% to 25%, and most preferably from about 0,2% to 20% by weight of the oral composition.

The composition reacts to a change in the biochemical environment, for instance a rapid dissolvement of ultra finely divided HA occurs when the pH is decreasing, that provides an active utilization of Ca and PO₄ - ions in the osteogenesis process: the size and configuration of the inventive crystals are adapted to the maximum to the dental enamel structure, which is mostly composed of HA, that suggests its use in the osteo-reparative process as a building material.

The ultra finely divided HA possesses a high adhesive-sorption activity to the dental enamel and to microdefects on its surface, that favour the preventive measures preventing the spread of caries disease and also possesses a high sorption activity in respect to proteins and aminoacids of paradentium tissues, that stimulates an active preventive treatment of the inflammable-destructive diseases such as paradenitis and paradentosis.

Moreover, the stomatic composition of the present invention will contain other conventional ingredients in addition to HA possessing osteo-reparative properties, whose introduction into the composition depends on the form of the product. For instance, in the case of an oral product in the form of dentifrice paste, cream or gel, the product will comprise a liquid phase containing humectants and binding thickeners which act to maintain the particulate solid abrasive and HA crystals in the form of stable suspension in the liquid phase.

Surfactants and flavouring agents are also usual ingredients for various inventive embodiments of oral compositions.

The humectants usually used are glycerol or sorbitol. However, other humectants may be used according to the invention including polyethyleneglycol, propyleneglycol, lactitol and hydrogenated corn syrop. The amount of humctant will generally range from about 0% to 85% by weight of product. The remainder of the liquid phase will consist substantially of water. The liquid phase can be water or a non-aqueous composition.

As binding agents and thickeners, various substances can be used such as sodium carboxymethylcellulose, sodium hydroxyethylcellulose and xanthan gum. Natural gum bindings can be included such as gum tragacanth, gum karaya of Irish moss, etc. Any mixture of binding agents and thickeners can be also used. The amount of bindings and thickeners usually included into the oral composition is in the range of 0% to 10% by weight of the oral composition.

Moreover, any materials as widely disclosed in the literature generally also might be used for the invention as surfactants, i.e. surfactants like sodium lauryl sulphate, dodecylbenzene sulphonate and sodium lauryl sarcosinate. Other anionic surfactants also can be used as well as cationic and amphoteric and non-ionic surfactants. Surfactants are generally present in the composition in the amount of 0% to 5% by weight of the oral composition.

Flavours that are generally used in the oral compositions are those based on oils of spearmint and peppermint and might be used for the invention. Examples of other flavouring materials used are menthol, clove, wintergreen, eucalyptus and aniseed. A preferable amount of flavours is from 0% to 5% by weight in respect to the oral composition.

As abrasive materials, silica dioxide of various modifications, aluminium oxide, calcium carbonate, dicalcium phosphate anhydrite, dicalcium phosphate dihydrate, sodium metaphosphate insoluble in water, and thereof mixtures may be used. The amount of abrasive materials ranges from 0.0% to 25%. The oral composition may include a wide variety of optional ingredients. These include antimicrobial and anti-plaque agents for example chlorhexidine or 2,4,4-trichloro-2hydroxy-diphenyl ether, or zink compounds (see EPA-161898) anti-tartar ingredients such as condensed phosphates, e.g. alkali et al pyrophosphates, hexametaphosphatesor polyphosphates (see US-A-4 515772 and US-A-4 627977) or zink citrates (see US-A-4 100269), sweetening agents such as saccharin. Preservatives such as formalin, sodium benzoate. colouring agents (for instance titanium dioxide) or pH-controlling agents, such as acid base or buffer agents the oral composition may also include agents enhancing the gingivitis system of the mouth cavity and representing extracts of various natural plants such as urtica, millefolium, chamomilla hypericum, salvia, etc. in the aqueous or aqueous-alcoholic forms.

The stomatic composition depending on its form (dentifrice paste, cream or gel) is maintained in contact with the tissue of the oral cavity from 15 sec to 12 hours.

The following examples of dentifrice pastes and gel comprising synthetic ultra finely divided HA possessing osteo-reparative properties as described above illustrate the invention. Percentages and parts of the components are by weight.

Belowstanding preferred embodiments of the invention are shown in its composition.

### Examples N1 and 2.

### Toothpaste prepared from the following ingredients.

| Ingredients, % | | |
|---|---|---|
| Example | 1 | 2 |
| Ultra finely divided Hydroxyapatite | 0,2 | 2,0 |
| Silica aerogel | 22,0 | 15,0 |
| Sodium carboxymethylcellulose | 1,0 | 1,0 |
| Glycerol distilled | 20,0 | 20,0 |
| Sorbitol | 20,0 | 17,0 |
| Titanium dioxide | 0,6 | 0,5 |
| Sodium benzoate | 0,4 | 0,6 |
| Aqueous-alcohol extract of chamomilla | 1,0 | 0,8 |
| Aqueous-alcohol extract of hypericum | 1,0 | 0,8 |
| Sodium saccharin | 0,1 | 0,06 |
| Flavour | 1,0 | 1,3 |
| Sodium lauryl sulphate | 1,5 | 1,5 |
| Water | to 100,0 | to 100,0 |

### Examples N 3 to 7

### Toothpaste prepared from the following ingredients.

| Ingredients,% | | | | | |
|---|---|---|---|---|---|
| Example | 3 | 4 | 5 | 6 | 7 |
| Ultra finely divided hydroxyapatite | 2,5 | 2,5 | 2,5 | 2,5 | 2,5 |
| Silica aerogel | 17,0 | 17,0 | 17,0 | 17,0 | 17,0 |
| Sodium hydroxyethylcellulose | 1,6 | - | - | 1,6 | - |
| Sodium carboxymethylcellulose | - | 1,1 | 1,1 | - | 0,9 |
| Sorbitol | 20,0 | 20,0 | 16,0 | 20,0 | 20,0 |
| Glycerol distilled | 20,0 | 20,0 | 20,0 | 20,0 | 20,0 |
| Polyethyleneglycol | - | - | 5,0 | - | - |
| Sodium lauryl sulphate | 1,5 | 1,5 | 1,5 | 1,5 | 1,5 |
| Tetrasodium pyrophosphate | - | 1,5 | - | - | - |
| Tetrapotassium pyrophosphate | - | - | - | 2,5 | - |
| Sodium trimetaphosphate | - | - | 2,0 | - | - |
| Zinc citrate trihydrate | - | - | - | - | 0,5 |
| Titanium dioxide | 0,6 | 0,6 | 0,6 | 0,6 | 0,6 |
| Sodium benzoate | 0,5 | 0,5 | 0,6 | - | - |
| Formalin | - | - | - | 0,05 | 0,05 |
| Aqueous-alcohol extract of salvia | 0,5 | 0,5 | - | - | - |
| Aqueous-alcohol extract of millefolium | 0,9 | 0,9 | 0,5 | 0,5 | - |
| Aqueous-alcohol extract of chamomilla | - | - | 1,0 | 0,7 | - |
| Triclosan | - | - | 0,2 | - | 0,2 |
| Sodium saccharin | 0,06 | 0,06 | 0,06 | 0,06 | 0,06 |
| Flavour | 1,0 | 1,0 | 1,0 | 1,0 | 1,0 |
| Water | | | in all example to 100,0 | | |

### Examples N8 and 9

### Gel preventing paradenitis.

| Ingredients,% | | |
|---|---|---|
| Example | 8 | 9 |
| Ultra finely divided hydroxyapatite | 5,0 | 4,0 |
| Sodium hydroxyethylcellulose | 2,0 | 2,5 |
| Silica aero | 5,0 | - |
| Glycerol distilled | 10,0 | - |
| Sorbitol | 25,0 | 45,0 |
| Sodium benzoate | 0,5 | - |
| Triclosan | - | 0,3 |
| Flavour | 0,2 | 0,15 |
| Sodium lauryl sulphate | 0,2 | 0,15 |
| Sodium saccharin | 0,07 | 0,07 |
| Water | to 100,00 | to 130,00 |

### Industrial application

The stomatic composition can be used to cure microdefects of the basic substance of the dental enamel, e.g. to prevent the spread of caries, and is also useful for preventive measures avoiding the spread of inflammable-destructive diseases of paradentium tissues, such as pardenitis and paradentosis.

The stomatic composition can be used in the form of tooth pastes, tooth creams and gels. Moreover, the composition can be included as a component in jewing gum, pastilles, tooth elixir and formulations to rinse mouth.

The stomatic composition according to the invention is capable to stimulate reparative osteogenesis processes and possessing a high bioactivity and specific pharmacological activity. Moreover, this composition is useful for combatting dental caries and to prevent the spread of such inflammable-destructive diseases of paradentium tissues as paradenitis and paradentosis, on the basis of hydroxyapatite also optionally comprising abrasive materials, humectants, thickeners, surfactants, flavouring agents, and a number of optional ingredients.

## Claims

1. A composition for stomatic applications **characterised in**
**that** it comprises particles of hydroxyapatite with an average particle size in length (1), width (d) and thickness (h) of: 1 from 0,2 µm to 0,01 µm, d from 0,1 µm to 0,001 µm, and h from 0,1 µm to 0,0001 µm.

2. The composition according to claim 1 **characterised in**
**that** it comprises particles of hydroxyapatite with an average particle size in length (1), width (d) and thickness (h) of l = 0,06 µm +/- 50 %, d = 0,015 µm +/- 50 % and h = 0,005 µm +/- 50 %.

3. The composition according to claim 1 **characterised in**
**that** it comprises particles of hydroxyapatite with an average particle size in length (1), width (d) and thickness (h) of l = 0,06 µm, d = 0,015 µm, h = 0,005 µm.

4. The composition according to one of claims 1 to 3 **characterised in**
**that** it comprises particles of hydroxyapatite having a specific surface of hydroxiapatite from 100 m²/g to 150 m²/g.

5. The stomatic composition according to one of claims 1 to 4 **characterized in**
**that** it comprises said hydroxyapatite particles ultra finely divided.

6. The composition according to one of claims 1 to 5 **characterised in that** the ultra finely divided hydroxyapatite particles are present in the composition in an amount of 0,1% to 50% by weight.

7. The composition according to one of claims 1 to 6 **characterised in that** the ultra finely, divided hydroxyapatite is a synthetic hydroxyapatite which contains 99,9% of Ca₁₀(PO₄)₆(OH)₂ by weight.

8. The composition according to one of claims 1 to 7 further **characterised by** at least one substance of the group consisting of
- humectants in a range from about 0% to 85% by weight,
- bindings and thickeners a range of 0% to 10% by weight,
- abrasive materials in a range from 0.0% to 25%,
- Surfactants in a range from 0% to 5% by weight,
- Flavours in a range from 0% to 5% by weight.

9. The composition according to one of claims 1 to 8 further **characterised by**
agents enhancing the gingivitis system of the mouth cavity and comprising extracts of natural plants including at least one of the group consisting of urtica, millefolium, chamomilla hypericum, salvia, etc. in the aqueous an in the aqueous-alcoholic form.

10. The composition according to one of claims 1 to 9 further **characterised by**
antimicrobial and anti-plaque agents.

## Patentansprüche

1. Zusammensetzung für Stomatika, insbesondere für die Anwendung im Mund- und Rachenraum,
**dadurch gekennzeichnet,**
**dass** diese Hydroxyapatit-Partikel mit einer mittleren Partikelgröße folgender Länge (l), Breite (b) und Dicke (h) umfasst: 1 von 0,2 µm bis 0,01 µm, b von 0,1 µm bis 0,001 µm und h von 0,1 µm bis 0,0001 µm.

2. Zusammensetzung nach Anspruch 1, **dadurch gekennzeichnet,**
**dass** diese Hydroxyapatit-Partikel mit einer mittleren Partikelgröße folgender Länge (l), Breite (b) und Dicke (h) umfasst: l = 0,06 µm +/- 50%, b = 0,015 µm +/- 50% und h = 0,005 µm +/- 50%.

3. Zusammensetzung nach Anspruch 1, **dadurch gekennzeichnet,**
**dass** diese Hydroxyapatit-Partikel mit einer mittleren Partikelgröße folgender Länge (l), Breite (b) und Dicke (h) umfasst: l = 0,06 µm, b = 0,015 µm und h = 0,005 µm.

4. Zusammensetzung nach einem der Ansprüche 1 bis 3, **dadurch gekennzeichnet,**
**dass** diese Hydroxyapatit-Partikel mit einer spezifischen Hydroxyapatit-Oberfläche von 100 m²/g bis 150 m²/g umfasst.

5. Zusammensetzung nach einem der Ansprüche 1 bis 4, **dadurch gekennzeichnet,**
**dass** diese Hydroxyapatit-Partikel ultrafein verteilt enthält.

6. Zusammensetzung nach einem der Ansprüche 1 bis 5, **dadurch gekennzeichnet,**
**dass** die ultrafein verteilten Hydroxyapatit-Partikel in der Zusammensetzung in einem Anteil von 0,1 bis 50 Gewichtsprozent vorhanden sind.

7. Zusammensetzung nach einem der Ansprüche 1 bis 6, **dadurch gekennzeichnet,**
**dass** die ultrafeinen, verteilten Hydroxyapatit-Partikel ein synthetisches Hydroxyapatit sind, welches 99,9 Gewichtsprozent Ca₁₀(PO₄)₆(OH)₂ enthält.

8. Zusammensetzung nach einem der Ansprüche 1 bis 7, ferner **gekennzeichnet durch** mindestens eine Substanz aus der Gruppe, bestehend aus:
- Wasserbindemitteln in einem Bereich von etwa 0 bis 85 Gewichtsprozent,
- Binde- und Verdickungsmitteln in einem Bereich von 0 bis 10 Gewichtsprozent,
- abrasiven Materialien in einem Bereich von 0% bis 25%,
- Tensiden in einem Bereich von 0 bis 5 Gewichtsprozent,
- Geschmacksstoffen in einem Bereich von 0 bis 5 Gewichtsprozent.

9. Zusammensetzung nach einem der Ansprüche 1 bis 8, ferner **gekennzeichnet durch** Mittel, welche das Zahnfleischsystem der Mundhöhle stärken und natürliche Pflanzenextrakte beinhalten, einschließlich mindestens eines der Gruppe, bestehend aus: Urtica, Millefolium, Chamomilla hypericum, Salvia, usw. in der wässrigen und in der wässrig-alkoholischen Form.

10. Zusammensetzung nach einem der Ansprüche 1 bis 9, ferner **gekennzeichnet durch** antimikrobielle und Anti-Plaque-Mittel.

## Revendications

1. Composition pour applications buccales,
**caractérisée en ce qu'**elle comprend des particules d'hydroxyapatite ayant des dimensions moyennes de particules consistant en une longueur (l), une largeur (d) et une épaisseur (h) suivantes : 1 de 0,2 µm à 0,01 µm, d de 0,1 µm à 0,001 µm, et h de 0,1 µm à 0,001 µm.

2. Composition suivant la revendication 1,
**caractérisée en ce qu'**elle comprend des particules d'hydroxyapatite ayant des dimensions moyennes de particules, consistant en une longueur (l), une largeur (d) et une épaisseur (h) suivantes : 1 = 0,06 µm ± 50 %, d = 0,015 µm ± 50 % et h = 0,005 µm ± 50 %.

3. Composition suivant la revendication 1,
**caractérisée en ce qu'**elle comprend des particules d'hydroxyapatite ayant des dimensions moyennes de particules consistant en une longueur (1), une largeur (d) et une épaisseur (h) suivantes : 1 = 0,06 µm, d = 0,015 µm, h = 0,005 µm.

4. Composition suivant une des revendications 1 à 3,
**caractérisée en ce qu'**elle comprend des particules d'hydroxyapatite ayant une surface spécifique de l'hydroxyapatite comprise dans l'intervalle de 100 m²/g à 150 m²/g.

5. Composition buccale suivant une des revendications 1 à 4, **caractérisée en ce qu'**elle comprend lesdites particules d'hydroxyapatite à l'état ultrafinement divisé.

6. Composition suivant une des revendications 1 à 5, **caractérisée en ce que** les particules d'hydroxyapatite ultra-finement divisées sont présentes dans la composition en une quantité de 0,1 % à 50 % en poids.

7. Composition suivant une des revendications 1 à 6, **caractérisée en ce que** l'hydroxyapatite ultrafinement divisée est une hydroxyapatite synthétique qui contient 99,9 % de Ca₁₀(PO₄)₆(OH)₂ en poids.

8. Composition suivant une des revendications 1 à 7, **caractérisée en outre par** au moins une substance du groupe consistant en :
- des agents mouillants en une quantité comprise dans l'intervalle d'environ 0 % à 85 % en poids,
- des liants et des épaississants en une quantité comprise dans l'intervalle de 0 % à 10 % en poids,
- des matières abrasives en une quantité comprise dans l'intervalle de 0,0 % à 25 %,
- des agents tensio-actifs en une quantité comprise dans l'intervalle de 0 % à 5 % en poids,
- des agents aromatisants en une quantité comprise dans l'intervalle de 0 % à 5 % en poids.

9. Composition suivant une des revendications 1 à 8, **caractérisée en outre par** la présence d'agents bénéfiques pour le système gingival de la cavité buccale et comprenant des extraits végétaux naturels, comprenant au moins un extrait d'une plante du groupe consistant en Urtica, Millefolium, Chamomilla, Hypericum, Salvia, etc., sous forme aqueuse et sous forme aqueuse-alcoolique.

10. Composition suivant une des revendications 1 à 9, **caractérisée en outre par** la présence d'agents anti-microbiens et anti-plaque.
